Europäisches Patentamt

European Patent Office

Office européen des brevets

(11) Publication number: **0 406 913 A1**

# EUROPEAN PATENT APPLICATION

(21) Application number: 90115259.5

(22) Date of filing: 03.08.84

(51) Int. Cl.5: **C12N 15/00**, C07H 21/04, C12P 21/02, C12N 1/20, //(C12N1/20,C12R1:19)

This application was filed on 08 - 08 - 1990 as a divisional application to the application mentioned under INID code 60.

(30) Priority: 10.08.83 US 521966
26.07.84 US 633451

(43) Date of publication of application:
**09.01.91 Bulletin 91/02**

(60) Publication number of the earlier application in accordance with Art.76 EPC: **0 135 094**

(84) Designated Contracting States:
**AT BE CH DE FR GB IT LI LU NL SE**

(71) Applicant: **AMGEN INC.**
**1840 Dehavilland Drive**
**Thousand Oaks California 91320(US)**

(72) Inventor: **Banks, Allen R.**
**2886 Middlefork Road Boulder**
**Colorado 80302(US)**
Inventor: **Peters, Mar A.**
**945 37th Street Boulder**
**Colorado 80303(US)**
Inventor: **Snitman, David L.**
**1475 Ithaca Drive Boulder**
**Colorado 80303(US)**

(74) Representative: **Brown, John David et al**
**FORRESTER & BOEHMERT**
**Widenmayerstrasse 4/14/I**
**D-8000 München 22(DE)**

(54) Microbial expression of insulin-like growth factor.

(57) Disclosed is the manufacture of DNA sequences comprising structural genes coding for (1) a polypeptide having the amino acid sequence and properties of insulin-like growth factor I; for (2) a polypeptide having the amino acid sequence and properties of insulin-like growth factor II; and for (3) polypeptide analogs of insulin-like growth factor I and insulin-like growth factor II which differ from the naturally-occurring forms in terms of the identity and/or location of one or more amino acids, e.g., [Thr$^{59}$] IGF-I and [Arg$^{54}$Arg$^{55}$] IGF-II.

EP 0 406 913 A1

# MICROBIAL EXPRESSION OF INSULIN-LIKE GROWTH FACTOR

## BACKGROUND

This is a continuation-in-part application of co-pending U.S. Patent Application Serial No. 521,966, filed August 10, 1983.

The present invention relates generally to the manipulation of genetic materials and, more particularly, to the manufacture of specific DNA sequences useful in recombinant procedures to secure the microbial expression of insulin-like growth factors I and II and of polypeptide analogs thereof.

Insulin-like growth factor I (IGF-I) and insulin-like growth factor II (IGF-II) are structurally-related, naturally-occurring polypeptides found in human serum. [Rinderknecht, E., et al., PNAS (USA) , Vol. 73, pages 2365-2369 (1976)]. IGF-I is a single-chain polypeptide of 70 amino acid residues cross-linked by three disulfide bridges, and having a calculated molecular weight of 7649. IGF-II has an amino acid sequence of 67 amino acid residues cross-linked by three disulfide bridges, and has a calculated molecular weight of 7471. [Rinderknecht, et al., J.Biol.Chem. , 253 , pages 2769-2776 (1978); Rinderknecht, et al., F.E.B.S. Letters , 89 , pages 283-286 (1978)]. IGF-I and IGF-II have a 62% structural homology, and both are structurally homologous to human proinsulin. [See, Hintz, et al., J.Clin. Endocrinology and Metabolism , 50 , pages 405-407 (1980)].

IGF-I and IGF-II have been classified as "somatomedins" on the basis of their biological actions and growth hormone dependence. The somatomedins are a family of small peptides, which includes somatomedin A, somatomedin C, insulin-like growth factors I and II, and multiplication stimulating activity factor. Considerable evidence has been obtained which indicates that IGF-I is identical to somatomedin C [Van Wyk, J.J., et al., J.Clin.Endocrinol.Metab. , 50 , pages 206-208 (1980); Hintz, R.L., et al., J.Clin.Endocrinol.Metab. , 50 , pages 405-407 (1980)]. Somatomedins share three major characteristics: they appear to contribute to the same biological actions; they appear to circulate bound to larger carrier proteins; and they originate at least in part in the liver rather than in a gland. Limited studies indicate that other tissues such as the kidney may also release somatomedins.

Somatomedins have many biological functions crucial to growth. Somatomedins have anabolic insulin-like actions on fat and muscle which cannot be suppressed by anti-insulin antibodies. The close correlation between somatomedin activity and insulin action suggests that insulin contributes to growth through somatomedins. Their growth-promoting effects include enhancement of cell multiplication and stimulation of cartilage proliferation, stimulation of transport of amino acids, synthesis of RNA, DNA and protein, and incorporation of sulfate into proteoglycan and of proline into collagen. Much mammalian post natal growth is due to stimulation of cartilage growth by somatomedins and growth in utero may also be somatomedin-dependent. [Phillips, L.S., et al., New England Journal of Medicine , Vol. 302, pages 438-446 (February 1980)].

Somatomedins also act to stimulate transport of amino acids and sugar and incorporation of glucose into glycogen in muscle, where the somatomedins are generally as potent as insulin. In adipose tissues, somatomedins stimulate sugar transport, glucose oxidation to carbon dioxide, and incorporation into lipids and inhibition of basal and epinephrin stimulated lipolysis.

Clinical observations have led to the hypothesis that somatomedins as the target products of growth hormone action have a negative feedback effect on growth hormone secretion. Low levels of somatomedins coexist with high levels of growth hormone in kwashiorkor, Laron-type dwarfism, liver disease, and in the newborn. Somatomedins are very low in growth hormone deficiency and levels are variable in ideopathic growth hormone deficiency. Children with partial growth hormone deficiencies have higher levels than those with complete deficiency. Causes of high somatomedin levels include agromegaly, pituitary gigantism, adolescence, somatomedin resistance syndrome, and obesity. Thus, somatomedin measurements may be useful in diagnosing growth diseases and monitoring the effect of therapy in growth hormone-related diseases.

Somatomedins in circulation may be measured by a variety of chemical and immunological assays which vary in simplicity, precision, and reliability. Significantly, assay procedures also vary in specificity and sensitivity due to the absence of a uniform somatomedin and/or anti-somatomedin antibody standards.

Clearly, the availability of large quantities of somatomedins such as IGF-I and IGF-II (as well as polypeptide analogs thereof) would greatly facilitate studies aimed at the elucidation of the biochemical mechanisms of growth control in humans and animals. Further, synthetic production of IGF-I and II in large quantities would permit long-term in vivo study and treatment of growth deficient states such as pituitary

2

dwarfism, as well as the study and treatment of repair processes such as wound healing.

Somatomedins have been isolated in very small quantities from large volumes of plasma or serum. A major problem in isolative processing has been achieving good yields of these peptides after initial extraction with acid ethanol. [Phillips, et al., New England J. Med. , 302 , pages 371-380 (1980)]. Recently, monoclonal antibodies against IGF-I have been generated by the hybridoma technique. [Laubli, et al., F.E.B.S. Letters , 49 , pp. 109-112 (1982)]. Attempts to isolate IGF-I from sera using the monoclonal antibody did not allow discrimination between IGF-I and IGF-II, possibly due to impurity of the initial IGF immunogen or to the specificity of the monoclonal to an antigenic determinant common to both IGF-I and IGF-II. The affinity purification procedures described by Laubli, et al., supra , also generated a large impurity of non-protein origin. Finally, attempts to effect the total chemical synthesis of IGF-I have resulted in low yields of pure material .[See, Li, et al., PNAS (USA) , 80 , pages 2216-2220 (1983)].

The use of recombinant DNA techniques for the manufacture, cloning and expression of structural genes for IGF-I and IGF-II was alluded to in an article appearing in Biotechnology News , Vol. 3, No. 10, pages 1-3 (May 15, 1983) and principally dealing with yeast secretion of numerous foreign proteins. Apparently, various structural genes were manufactured or obtained by cDNA procedures, cloned, and then joined to the leader sequence of the yeast alpha factor precursor gene. No details were provided on the method of gene synthesis or on analysis of the purity, yield, or biological activity of the secreted products. Nor were polypeptide analogs of IGF-I and II mentioned.

There thus continues to be a need in the art for fully operative methods and materials suitable for use in the large-scale production of pure, biologically active IGF-I and IGF-II and polypeptide analogs thereof.

## BRIEF SUMMARY

Provided by the present invention are manufactured genes capable of directing synthesis, in a selected host microorganism, of human insulin-like growth factor I and insulin-like growth factor II. In preferred forms of manufactured genes, the base sequence includes one or more codons selected from among alternative codons specifying the same amino acid on the basis of preferential expression characteristics for the codon in a projected host microorganism, e.g., E.coli . Other preferred forms of manufactured genes include those wherein there is provided a base codon specifying an additional amino acid residue in the polypeptide coded for which facilitates the direct expression in E.coli organisms (e.g., an initial Met residue). In still other preferred forms of manufactured genes, the sequence of base codons specifying the desired polypeptide is preceded by and/or followed by and/or includes one or more sequences of bases facilitating formation of expression vectors or generation of new structural genes for polypeptide analogs, i.e., sequences of bases providing for selected restriction endonuclease cleavage sites on one or both ends of the structural gene or at intermediate positions therein.

Also provided by the present invention are: (1) manufactured genes capable of directing the microbial expression of insulin-like growth factor polypeptide analogs which differ from the insulin-like growth factors in terms of the identity and/or location of one or more amino acid residues (e.g., [Thr$^{59}$] IGF-I and [Arg$^{54}$Arg$^{55}$] IGF-II); and (2) fusion genes comprising manufactured genes according to the invention fused to a second gene capable of directing synthesis of a second polypeptide (e.g., $\beta$-galactosidase) in a manner permitting the microbial expression of a fused polypeptide including insulin-like growth factor polypeptide or an insulin-like growth factor analog.

In practice of the invention to generate polypeptide products, manufactured DNA sequences are inserted into viral or circular plasmid DNA vectors to form hybrid vectors and the hybrid vectors are employed to transform host microorganisms such as bacteria (e.g., E.coli ) or yeast cells. The transformed microorganisms are thereafter grown under appropriate nutrient conditions and express the polypeptide products of the invention.

Other aspects and advantages of the present invention will be apparent upon consideration of the following detailed description thereof.

## DETAILED DESCRIPTION

As employed herein, the term "manufactured" as applied to a DNA sequence or gene shall designate a product either totally chemically synthesized by assembly of nucleotide bases or derived from the biological replication of a product thus chemically synthesized. As such, the term is exclusive of products "synthesized" by cDNA methods or genomic cloning methodologies which involve starting materials which are initially of biological origin.

The following abbreviations shall be employed herein to designate amino acids: Alanine, Ala; Arginine, Arg; Asparagine, Asn; Aspartic acid, Asp; Cysteine, Cys; Glutamine, Gln; Glutamic acid, Glu; Glycine, Gly; Histidine, His; Isoleucine, Ile; Leucine, Leu; Lysine, Lys; Methionine, Met; Phenylalanine, Phe; Proline, Pro; Serine, Ser; Threonine, Thr; Tryptophan, Trp; Tyrosine, Tyr; Valine, Val. The following abbreviations shall be employed for nucleotide bases: A for adenine; G for guanine; T for thymine; U for uracil; and C for cytosine.

For ease of understanding of the present invention, Table I below provides a tabular correlation between the 64 alternate triplet nucleotide base codons of DNA and the 20 amino acids and transcription termination ("stop") function specified thereby.

TABLE I

| FIRST POSITION | SECOND POSITION | | | | THIRD POSITION |
|---|---|---|---|---|---|
| | T | C | A | G | |
| T | Phe | Ser | Tyr | Cys | T |
| | Phe | Ser | Tyr | Cys | C |
| | Leu | Ser | Stop | Stop | A |
| | Leu | Ser | Stop | Trp | G |
| C | Leu | Pro | His | Arg | T |
| | Leu | Pro | His | Arg | C |
| | Leu | Pro | Gln | Arg | A |
| | Leu | Pro | Gln | Arg | G |
| A | Ile | Thr | Asn | Ser | T |
| | Ile | Thr | Asn | Ser | C |
| | Ile | Thr | Lys | Arg | A |
| | Met | Thr | Lys | Arg | G |
| G | Val | Ala | Asp | Gly | T |
| | Val | Ala | Asp | Gly | C |
| | Val | Ala | Glu | Gly | A |
| | Val | Ala | Glu | Gly | G |

The manufacture of structural genes according to the present invention is preferably carried out according to the procedures disclosed in the co-owned, co-pending U.S. Patent Application Serial No. 375,493, filed May 6, 1982 (corresponding PCT Application No. 83/04029, published November 24, 1983) by Yitzhak Stabinsky, entitled "Manufacture and Expression of Structural Genes", which application is incorporated by reference herein for the purpose of describing the steps of the procedure.

The following examples illustrate practice of the invention in the manufacture of the DNA sequences coding for microbial expression of IGF-I, IGF-II and polypeptide analogs thereof. Also illustrated is the construction of expression vectors for direct expression of desired polypeptides and expression of fused polypeptides including desired polypeptides.

The following example is directed to the general procedure employed in the synthesis of oligonucleotide fragments employed to manufacture structural genes according to the invention.


EXAMPLE 1


Oligonucleotide fragments were synthesized using a three-step procedure and several intermediate washes. Polymer bound dimethoxytrityl protected nucleoside in a sintered glass funnel was first stripped of its 5'-protecting group (dimethoxytrityl) using 3% trichloroacetic acid in dichloromethane for 1-1/2 minutes. The polymer was then washed with dichloromethane, methanol and acetonitrile. The washed polymer was

4

then rinsed with dry acetonitrile, placed under argon and then treated in the condensation step as follows. 0.5 ml of a solution of 10 mg tetrazole in acetonitile was added to the reaction vessel containing polymer. Then 0.5 ml of 30 mg protected nucleoside phosphoramidite in acetonitrile was added. This reaction was allowed to react for 2 minutes. The reactants were then removed by suction and the polymer rinsed with acetonitrile. This was followed by the oxidation step wherein 1 ml of a solution containing 0.1 molar $I_2$ in 2-6-lutidine/$H_2$O/THF, 1:2:2, was reacted with the polymer bound oligonucleotide chain for 2.5 minutes. Following a methanol, THF, and dichloromethane wash, the cycle began again with a trichloroacetic acid in $CH_2Cl_2$ treatment. The cycle was repeated until the desired oligonucleotide sequence was obtained.

The final oligonucleotide chain was treated with thiophenol dioxane, triethylamine 1:2:2, for 45 minutes at room temperature. Then, after rinsing with methanol and diethylether, the oligonucleotide was cleaved from the polymer with concentrated ammonia at room temperature. After decanting the solution from the polymer, the concentrated ammonia solution was heated at 60°C for 16 hours in a sealed tube.

Each oligonucleotide solution was then extracted four times with 1-butanol. The solution was loaded into a 20% polyacrylamide 7 molar urea electrophoresis gel and, after running, the appropriate product band was isolated.

The following example is directed to the manufacture of structural genes coding for IGF-I and also illustrates the manner in which structural genes for polypeptide analogs thereof may be prepared.

EXAMPLE 2

Eighteen specific deoxyoligonucleotides (numbered 1 through 18) were synthesized according to the procedures of Example 1. The oligonucleotides were purified by polyacrylamide gel electrophoresis and were phosphorylated at the 5' ends using ATP and $T_4$ polynucleotide kinase in a standard reaction using one nanomole of DNA, a two fold excess of ATP and 1 unit of $T_4$ kinase in 20 $\mu$l of buffer made with 50 mM hydroxyethylpiperazine ethane sulfonic acid, 10 mM $MgCl_2$, 10 mM dithiothreitol, pH 7.6. After reaction, the kinase was destroyed by boiling for 5 minutes. These phosphorylated oligonucleotides in the buffer were then used directly for ligation.

The oligonucleotides in 20 $\mu$l standard buffer were combined to form short duplexes. Each duplex was formed by combining two complementary sequences in equimolar amounts, boiling the mixture, then slow cooling over a 1/2 hour period to room temperature. In this way, nine duplexes were formed.

These nine duplexes were combined sequentially, annealing each set of duplexes at 37°C for 5 minutes until the final structural gene was in a single tube ready for ligation. The oligonucleotide mixture was then made 150 $\mu$molar in ATP and treated with 84 units of $T_4$ DNA ligase for 16 hours at 4°C. The fully ligated structural gene was then purified by polyacrylamide gel electrophoresis.

Table II below illustrates the finally assembled structural gene coding for direct microbial expression of IGF-I.

## TABLE II

```
              -1    1    2    3    4    5    6    7    8    9
    BamHI    Met  Gly  Pro  Glu  Thr  Leu  Cys  Gly  Ala  Glu
           ┌──────────────────── 1 ─────────────────┐ ┌────────
  5'-G ATC  CCT  ATG  GGT  CCG  GAA  ACA  CTG  TGC  GGC  GCT  GAA
     3'- GGA  TAC  CCA  GGC  CTT  TGT  GAC  ACG  CCG  CGA  CTT
        └──────────────────────────── 2 ──────────────────┘
```

TABLE II (cont'd)

| 10 | 11 | 12 | 13 | 14 | 15 | 16 | 17 | 18 | 19 | 20 | 21 | 22 |
|-----|-----|-----|-----|-----|-----|-----|-----|-----|-----|-----|-----|-----|
| Leu | Val | Asp | Ala | Leu | Gln | Phe | Val | Cys | Gly | Asp | Arg | Gly |

```
                ┌─────────3─────────┐ ┌───────────────────5────┐
CTG GTT GAC GCT CTG CAG TTT GTA TGC GGC GAC CGT GGT
GAC CAA CTG CGA GAC GTC AAA CAT ACG CCG CTG GCA CCA
        └─────────────────4─────────────────┘
```

| 23 | 24 | 25 | 26 | 27 | 28 | 29 | 30 | 31 | 32 | 33 | 34 | 35 |
|-----|-----|-----|-----|-----|-----|-----|-----|-----|-----|-----|-----|-----|
| Phe | Tyr | Phe | Asn | Lys | Pro | Thr | Gly | Tyr | Gly | Ser | Ser | Ser |

```
        ┌───────────7───────────┐ ┌─────────────────┐ ┌───────
 TTC TAT TTT AAC AAG CCG ACT GGC TAC GGT TCC TCT TCC
 AAG ATA AAA TTG TTC GGC TGA CCG ATG CCA AGG AGA AGG
└─────────6─────────┘           └───────────8───────────┘
```

| 36 | 37 | 38 | 39 | 40 | 41 | 42 | 43 | 44 | 45 | 46 | 47 | 48 |
|-----|-----|-----|-----|-----|-----|-----|-----|-----|-----|-----|-----|-----|
| Arg | Arg | Ala | Pro | Gln | Thr | Gly | Ile | Val | Asp | Glu | Cys | Cys |

```
└──9──┘ ┌─────────┐ ┌───────────11───────────┐ ┌───────
CGC CGT GCT CCA CAG ACT GGT ATC GTT GAT GAA TGC TGT
GCG GCA CGA GGT GTC TGA CCA TAG CAA CTA CTT ACG ACA
└───────────10───────────┘ └───────────12───────────┘
```

| 49 | 50 | 51 | 52 | 53 | 54 | 55 | 56 | 57 | 58 | 59 | 60 | 61 |
|-----|-----|-----|-----|-----|-----|-----|-----|-----|-----|-----|-----|-----|
| Phe | Arg | Ser | Cys | Asp | Leu | Arg | Arg | Leu | Glu | Met | Tyr | Cys |

```
└───────13───────┐ ┌───────────────15───────────┐
TTT CGT TCT TGC GAT CTG CGC CGT CTG GAA ATG TAT TGT
AAA GCA AGA ACG CTA GAC GCG GCA GAC CTT TAC ATA ACA
└───────────14───────────┘ └───────────16───────┘
```

| 62 | 63 | 64 | 65 | 66 | 67 | 68 | 69 | 70 | | | | SalI |
|-----|-----|-----|-----|-----|-----|-----|-----|-----|--|--|--|------|
| Ala | Pro | Leu | Lys | Pro | Ala | Lys | Ser | Ala | | | | |

```
└─┐ ┌───────────────17───────────┐ ┌───┐
GCT CCT CTG AAA CCC GCA AAG TCC GCT TAG TAG-3'
CGA GGA GAC TTT GGG CGT TTC AGG CGA ATC ATC AGC T-5'
└───────────────────18───────────────────┘
```

Included in the Table is the numbered sequence of amino acid residues specified when the DNA sequence is suitably inserted in the proper reading frame within a microbial expression vector. The polypeptide sequence is thus seen to comprise the series of seventy amino acids of naturally-occurring IGF-I together with an initial methionine residue (at position -1).

In the Table, codons specifying each amino acid are aligned below the designation of the residue specified. Bracketed regions in the DNA sequence indicate the eighteen separate oligonucleotides initially formed and also designate the intermediate duplexes (e.g., 1 and 2, 3 and 4, etc.). Wherever consistent with the procedures for construction of the manufactured gene, codon usage is selected on the basis of "preferences" of the projected expression host (e.g., E.coli ) according to the data set out in Grantham, et al., Nucleic Acids Research , 8 , pages r49-r62 (1980); Grantham, et al., Nucleic Acids Research , 8 , pages 1893-1912 (1980); and Grantham, et al., Nucleic Acids Research , 9 , pages r43-r74 (1981). See also, Bennetzen, et al., J.Biol.Chem. , 257 , pp. 3026-3031 (1982); and Grosjean, et al., Gene , 18 , pp. 199-209 (1982).

While the codons employed in generating the IGF-I structural gene of Table II are among those presently preferred, it will be understood that numerous changes may be made which may facilitate expression and/or facilitate manipulations involved in generating structural genes coding for IGF-I analogs. As an example of the former, use of an alternative proline-specifying codon at amino acid position 2 may improve ribosome binding of mRNA transcribed from the sequence illustrated. A suitable alternative to the -CCG- codon shown in the Table is -CCA- and this alternative can easily be provided by changing a single base during construction of each of oligonucleotides numbers 1 and 2. As an example of the latter, unique restriction enzyme recognition sites may be introduced into the DNA sequence without altering the amino acid sequence coded for, e.g., changing the pair of codons respectively specifying glutamic acid and leucine at positions 9 and 10 in oligonucleotide numbers 3 and 4 to -GAG CTC- will serve to introduce a Sall site; changing the codons for Gly[19] in oligonucleotide numbers 5 and 6 to -GGT- will complete the base sequence for a BstEll site; and changing the Leu[57] and adjacent Glu[58] codons in oligonucleotide numbers 15 and 16 to -CTC GAG-will generate a unique Xhol site.

Codon changes may also be employed to minimize potentially undesirable secondary structure present in the manufactured structural gene. As one example, the sequence of Table II was analyzed with the assistance of a computer program [Intelligenetics, Inc., SEQ-DNA Sequence Analysis System Reference Manual , TOPS 20 Version 3.0, p. 43 (1982)] to determine probable potential areas of unwanted secondary structure in the DNA itself and the RNA sequence transcribed from the DNA sequence. Based on the results of this analysis, the following illustrative codon changes may be made. The codon -GTT- at amino acid position 11 coding for valine, may be altered to -GTA- by changing one base in oligonucleotide numbers 3 and 4 of Table II. Similarly, the codon at amino acid position 12, coding for valine, may be altered by changing one base in oligonucleotide number 3 (GAC to GAT) and the corresponding base in oligonucleotide number 4 (GTC to ATC). These two codon changes minimize the predicted secondary structure of the manufactured gene coding for IGF-I.

At that end of the gene which specifies the amino terminal residues of the polypeptide (i.e., designated by the "5'" on the top strand) there is provided the sticky end of a selected restriction endonuclease enzyme recognition site (here, illustratively, a BamHI sticky end). At the end of the gene which specifies the carboxy terminal residues of the polypeptide (Ala[70]) there are provided translation stop codons (e.g., TAG, TAG) and a sticky end of a similarly selected restriction endonuclease recognition site (here, illustratively, a Sall sticky end). It will be understood that one or more entire, duplex, total recognition sites may be provided at each end which would be enzyme treated in the course of insertion in a vector.

Novel IGF-I analog genes coding for polypeptide sequences different from the natural sequence were also prepared. This was done by altering the base sequences of selected oligonucleotides and repeating the ligation step to obtain the new genes. By altering oligonucleotide numbers 15 and 16, the methionine at residue 59 was changed to threonine. The codon modification in oligonucleotide number 15 was from ATG to ACC. The modification in oligonucleotide number 16 was from CAT to GGT. If cyanogen bromide is employed to remove a Met[-1] residue, this change will prevent cleavage of the polypeptide at residue 59 and may produce a peptide with novel biological characteristics. It may be noted, for example, that replacement of Met [59] by Thr[59] appears by computer analysis to alter the hydropathicity of the polypeptide in that region.

Similarly according to computer analysis, by altering oligonucleotide numbers 15 and 16 and changing the methionine at residue 59 to valine or leucine, the hydropathicity appears to remain unchanged. The codon modifications in oligonucleotide number 15 are from ATG to GTG or TTG, respectively. In oligonucleotide number 16, the complementary codon changes are from CAT to CAC or CAA, respectively.

By altering oligonucleotide numbers 9 and 10, the alanine at residue 38 may be changed to valine. The modification in oligonucleotide number 9 is from GCT to GTT; the modification in ologonucleotide number 10 is from AGC to ACC. This modification in the IGF-I sequence would eliminate the naturally-occurring base labile sequence of Arg[36] Arg[37] Ala[38], which is unstable at high pH and may contribute difficulties in isolating IGF-I from a microbial host.

The following example is directed to the manufacture of structural genes coding for IGF-II and also illustrates the manner in which structural genes for polypeptide analogs may be prepared.

## EXAMPLE 3

Eighteen deoxyoligonucleotides were synthesized and combined to form duplexes which were sequentially annealed according to the general procedures of Examples 1 and 2 to yield the structural gene set out in Table III, below,

7

## TABLE III

```
              -1    1    2    3    4    5    6    7    8    9   10
       BamHI  Met  Ala  Tyr  Arg  Pro  Ser  Glu  Thr  Leu  Cys  Gly
       ┌────────────────────1───────────┬──────────3──────────┐
5'-GAT CCT  ATG  GCA  TAT  AGA  CCA  TCT  GAA  ACA  CTG  TGC  GGC
   ·3'-GA   TAC  CGT  ATA  TCT  GGT  AGA  CTT  TGT  GAC  ACG  CCG
       └───────────────2───────────┘              └─────────4──
```

```
11   12   13   14   15   16   17   18   19   20   21   22   23
Gly  Glu  Leu  Val  Asp  Thr  Leu  Gln  Phe  Val  Cys  Gly  Asp
┌────────────────5───────────────┐ ┌─────────7─────────┐
GGT  GAA  CTG  GTT  GAC  ACT  CTG  CAG  TTT  GTA  TGC  GGC  GAC
CCA  CTT  GAC  CAA  CTG  TGA  GAC  GTC  AAA  CAT  ACG  CCG  CTG
└────────────────────6───────────┘
```

```
24   25   26   27   28   29   30   31   32   33   34   35   36
Arg  Gly  Phe  Tyr  Phe  Ser  Arg  Pro  Ala  Ser  Arg  Val  Ser
┌─────────────────────┐ ┌──────────9──────────────────┐
CGT  GGT  TTC  TAT  TTT  AGC  CGT  CCG  GCT  TCT  CGC  GTA  TCC
GCA  CCA  AAG  ATA  AAA  TCG  GCA  GGC  CGA  AGA  GCG  CAT  AGG
└──────────8──────────┘              └──────────10──────────┘
```

```
37   38   39   40   41   42   43   44   45   46   47   48   49
Arg  Arg  Ser  Arg  Gly  Ile  Val  Glu  Glu  Cys  Cys  Phe  Arg
┌──────────────11───────────────┐ ┌──────────13──────────┐
CGT  CGC  TCT  CGT  GGT  ATC  GTT  GAG  GAA  TGC  TGT  TTT  CGT
GCA  GCG  AGA  GCA  CCA  TAG  CAA  CTC  CTT  ACG  ACA  AAA  GCA
└──────────┘ └─────────────12──────────────┘
```

```
50   51   52   53   54   55   56   57   58   59   60   61   62
Ser  Cys  Asp  Leu  Ala  Leu  Leu  Glu  Thr  Tyr  Cys  Ala  Thr
┌─────────────────────┐ ┌──────────────15───────────────┐ ┌──────
TCT  TGC  GAT  CTG  GCT  CTG  CTG  GAA  ACC  TAT  TGT  GCT  ACT
AGA  ACG  CTA  GAC  CGA  GAC  GAC  CTT  TGG  ATA  ACA  CGA  TGA
└──────────14──────────┘ └──────────────────16──────────────┘
```

```
63   64   65   66   67
Pro  Ala  Lys  Ser  Glu              SalI
┌──────────17──────────┐
CCC  GCA  AAG  TCC  GAA  TAG  TAG-3'
GGG  CGT  TTC  AGG  CTT  ATC  ATC  AGC  T-5'
└──────────────18──────────────┘
```

Included in the Table is the numbered sequence of amino acid residues specified when the DNA sequence is suitably inserted in the proper reading frame within a microbial expression vector. The polypeptide sequence thereby comprises the series of sixty-seven amino acids of naturally-occurring IGF-II with an initial methionine residue (at position -1).

The sequence is interpreted in the same way as previously described for the sequence of Example 2, Table II. Similarly, codon usage may be based on the "preferences" of the potential host microorganism, and changes may be made in the codons employed in the sequence to facilitate expression and/or manipulations involved in generating genes coding for IGF-II analogs, i.e., to insert internal restriction enzyme recognition sites into the DNA sequence.

At the 5' end of the sequence are bases forming the "sticky end" of a selected restriction endonuclease enzyme recognition site (here, BamHI). Transcription termination codons (e.g., TAG, TAG) and the sticky end of a similarly selected restriction endonuclease recognition site (here, e.g., SalI) are provided after the carboxy-terminal residue of the polypeptide (Glu[67]).

A novel IGF-II analog structural gene may be prepared by altering oligonucleotide numbers 14 and 15, to change the alanine and leucine residues at positions 54 and 55 to arginine residues such as are present in corresponding positions in IGF-I. The analog coded for by this sequence, [Arg$^{54}$,Arg$^{55}$] IGF-II, would thus incorporate residues at the region spanning residues 45 to 57 exactly duplicating the region spanning residues 46 through 58 of IGF-I. The codon modifications in oligonucleotide number 15 were from -GCT-CTG- to -CGT-CGG-; corresponding modifications in oligonucleotide number 14 were from -CAG-AGC- to -CCG-ACG-.

Another exemplary IGF-II analog gene may be constructed to incorporate the "C-peptide" region of the IGF-I gene DNA sequence into the IGF-II DNA sequence. Replacement of IGF-II amino acid residues 33 through 40, the C-peptide region, is effected by alterations in oligonucleotide numbers 9, 10, 11 and 12. The new oligonucleotides inserted into the IGF-II sequence specify the C-peptide region of IGF-I, i.e., amino acid residues 30 through 41. The analog is readily manufactured with new oligonucleotides 9′, 10′, 11′ and 12′, as below, replacing original oligonucleotides 9, 10, 11 and 12, respectively, in the construction illustrated in Table III.

```
    Ser Arg Pro Ala Gly Tyr Gly Ser Ser Ser Arg Arg Ala Pro
    ───────────────────9'──────────────────────   ───────────
    AGC CGT CCG GCT GGC TAC GGT TCC TCT TCC CGC CGT GCT CCA
        A GGC CGA CCG ATG CCA AGG AGA AGG GCG GCA CGA GGA
          ──────────────────────10'──────────────────────

    Gln Thr Gly Ile Val Glu
    ────11'──────────────────.
    CAG ACT GGT ATC GTT GAG
    GTC TGA CCA TAG CAA CTC CTT A
          ──────────12'──────────────
```

It will be noted that this IGF-II analog, designated IGF-II-C-I, includes more amino acid residues than the naturally-occurring polypeptide.

The following example relates to procedures for construction of expression vectors for use in transformation of microbial host cells to secure expression of IGF-I polypeptide products of the invention.


EXAMPLE 4


A. Construction of Plasmid pT5-4-IGF-I


The 226 base pair manufactured IGF-I gene illustrated in Table II of Example 2 and having BamHI and SalI sticky ends was inserted into the E.coli cloning vector pBR322 digested with BamHI and SalI . Clones with the gene were characterized by polyacrylamide gel electrophoresis to verify the estimated molecular weight for the IGF-I structural gene. ·

To further characterize the cloned manufactured DNA oligonucleotide, the 226 base pair fragment was excised from the pBR322 plasmid and inserted into bacteriophage M13mp8 and M13mp9 replicative form DNA at the BamHI and SalI sites. Clones with the inserted DNA in a defined orientation were isolated and characterized by polyacrylamide gel electrophoresis. Single-strand DNAs for one orientation were isolated and the DNA sequence for the IGF-I structural gene was verified using the Sanger Dideoxy sequencing technique. The manufactured gene for IGF-I was excised from the M13mp8 replicative form by treatment with restriction endonucleases BamHI and SalI .

The gene was next placed under control of a synthetic hybrid promoter/operator, T5A lac , in the plasmid pT5-4. The T5A-lac promoter/operator sequence is illustrated below:

HindIII

```
          ┌──────────1─────────┐ ┌────────3──────────────┬──────────
5'-AGCTTCATA AAAATTTTAG TTGCTTAATG CTAAAATTCT TGATATAATA
3'     AGTAT TTTTAAAATC AACGAATTAC GATTTTAAGA ACTATATTAT
          └──────────2───────────────────────4───────────┴────────
```

```
    ┌─────5─────────┬─────────7──────────────┐
TTCTCAATTG TGAGCGGATA ACAATTTATC TAAGGAG-3'
AAGAGTTAAC ACTCGCCTAT TGTTAAATAG ATTCCTCCTAG-5'
    └─────6─────────────────────┘ └──────8─────────
                                          BamHI
```

This 86-base pair promoter/operator sequence contains the T5A replica, a 50-base pair synthetic T5 early promoter sequence, designed based on the currently known sequences of the T5 early promoter, P25, P26, P28 and P207 [See, Bujard, TIBS , 5 : 274-278 (1980); Bujard, et al., pages 121-140 in Promoters: Structure and Function (R.Rodriguez, et al., eds.), Praiger, New York (1982)], and a 21-base pair lac - operator control se quence. The transcriptional activity of the promoter sequence is thereby minimally modulated approximately 200-fold by lac repressor enzyme. The lac operator sequence is positioned at the expected mRNA start site, as is found in the naturally-occurring lactose operon [see, Dickson, et al., Science , 182 : 27-35 (1975)].

Plasmid pT5-4 was constructed by inserting into the large fragment of the E.coli cloning vector pBR325 digested with the restriction endonuclease enzymes, HindIII and BamHI , the T5A-lac promoter/operator DNA sequence having HindIII and BamHI sticky ends. Clones with the promoter/operator sequence designated "T5-4" were characterized by molecular weight sizing of restriction fragments on PAGE and dideoxynucleotide DNA sequencing.

Plasmid pT5-4 was treated with restriction endonucleases BamHI and SalI and bacterial alkaline phosphatase, thereby cleaving the plasmid 3' to the promoter/operator. The excised manufactured IGF-I gene is then inserted into the plasmid between the BamHI and SalI restriction sites and ligated, creating plasmid pT5-4-IGF-I. The insertion of the 226 base pair oligonucleotide of IGF-I in the correct orientation has been verified by restriction enzyme analysis and molecular weight sizing using polyacrylamide gel electrophoresis.

## B. Construction of Plasmid pADP223

Upon association of the manufactured IGF-I structural gene with a suitable promoter/operator within plasmid pT5-4-IGF-I, further manipulations were performed in an attempt to generate an expression vector which would provide for high levels of E.coli expression of the desired protein. These manipulations involved use of a plasmid which is the subject of co-owned, co-pending, concurrently-filed U.S. Patent Application Serial No. 521,964, by Morris, entitled "DNA Vector". The disclosures of said application are specifically incorporated by reference herein. Briefly noted, plasmid pCFM414 (A.T.C.C. 40076), employed in the construction, includes a temperature-sensitive mutation in the copy control region. Upon transformation with this vector, host cell cultures grown at 34° C will have a low number of copies of the plasmid. The plasmid copy number increases fiftyfold (i.e., "runs away") within the host cells upon elevation of the culture temperature above 34° C. Growth at 37° C will ordinarily be lethal to the cells.

The specific manipulations involved in construction of a vector using A.T.C.C. 40076 were as follows. Plasmid pT5-4-IGF-I was digested with HindIII and SalI to generate a 303 base pair fragment including the IGF-I structural gene and the associated T-5 lac promoter/operator. Plasmid pCFM414 was digested with HindIII and XhoI and the large fragment was isolated. The HindIII / SalI fragment containing the IGF-I gene was then ligated with the large HindIII / XhoI fragment of pCFM414 to generate plasmid pADP223. While this construction retained an intact HindIII recognition site, ligation of the complementary sticky ends of SalI and XhoI did not restore either recognition site. It is also noteworthy that this construction resulted in the placement of the IGF-I structural gene 5' to transcription terminator sequence, Toop present 3' to the XhoI site in pCFM414.

The following example illustrates construction of an expression vector for use in transformation of microbial host cells to secure expression of [Thr$^{59}$] IGF-I as a fragment of a fusion protein, 8-galactosidase-

[Thr$^{59}$] IGF-I.

## EXAMPLE 5

The 226 base pair [Thr$^{59}$] IGF-I analog gene (hereafter "IGF-IT"), whose manufacture is described in Example 2, was inserted into E.coli cloning vector pBR322 using the BamHI and Sall restriction endonuclease sites. Resulting clones with the gene were characterized by polyacrylamide gel electrophoresis to verify the estimated molecular eight for the IGF-IT structural gene.

To further characterize the cloned synthetic DNA oligonucleotide, the 226 base pair fragment was excised from the pBR322 plasmid and inserted into bacteriophages M13mp8 and M13mp9 replicative form DNA at the BamHI and Sall sites. Clones with the inserted DNA in a defined orientation were isolated and characterized by polyacrylamide gel electrophoresis. Single-strand DNA for one orientation were isolated and the DNA sequence for the IGF-I structural gene was verified using the Sanger Dideoxy sequencing technique.

The IGF-IT gene was excised as a BamHI , Sall fragment from M13 bacteriophage replicative form DNA and inserted into a suitable plasmid (e.g., BamHI / Sall digested plasmid pTP) for amplification. Plasmid pTP has an EccoRI restriction endonuclease recognition site and an Xbal restriction endonuclease recognition site located 5' to its BamHI restriction endonuclease recognition site, at which the IGF-IT coding sequence is inserted. The DNA sequence as set out below from the EcoRI site to the BamHI site in pTP is the following:

```
     EcoRI   XbaI
5'-AA TTC TCT AGA ATG AAG AAA TAT TG-3'
3'-     G AGA TCT TAC TTC TTT ATA ACC TAG-5'
                                    BamHI
```

Thus there is provided in the resulting amplification plasmid, pTP-IGF-IT, an adenosine-rich series of bases prior to the IGF-IT polypeptide specifying sequences. The IGF-IT gene coding sequence, together with the adenosine-rich sequence, was then excised from the amplification plasmid with EcoRI and Sall and inserted into an EcoRI / Sall digested plasmid pBtra, which has a KpnI site 3' to the Sall restriction site. The resulting vector was designated pADP6.

pADP6 is then digested with restriction endonucleases to obtain the IGF-IT gene as an EcoRI / KpnI fragment. This fragment is inserted into an EcoRI / KpnI endonuclease cleaved plasmid p41 [Cheng, Y., et al., Gene , 14 , page 121 (1981)], which contains an EcoRI restriction site at codon 1004 of the β-galactosidase enzyme gene. The resulting plasmid vector, pADP201, contains the natural promoter/operator of β-galactosidase, the first 1004 codons of β-galactosidase, followed by the codons from the EcoRI site to the ATG codon of PTP-IGF-IT, followed by a methionine-specifying codon and the seventy codons of the IGF-IT gene.

Plasmid pADP201, when employed as an expression vector in a host microorganism, specifies expression of a 1084 amino acid 8-galactosidase-IGF-IT fusion peptide including the following sequence of amino acids: NH$_2$-[β-galactosidase (initial 1004 amino acids)]-Ser$^{-10}$-Arg$^{-9}$-Met$^{-8}$-Lys$^{-7}$-Lys$^{-6}$-Tyr$^{-5}$-Trp$^{-4}$-Ile$^{-3}$-Pro$^{-2}$-Met$^{-1}$ [IGF-IT (70 amino acids)]-COOH.

The following example relates to vectors for direct expression of [Thr$^{59}$] IGF-I with a short "leader" or "pro" sequence of residues at its amino terminal.

## EXAMPLE 6

The IGF-IT polypeptide may also be directly expressed as a peptide having an eight amino acid leader. Plasmid pTP-IGF-IT, as described in Example 5, is cleaved with endonucleases Xbal and Sall , resulting in a Xbal / Sall fragment containing an adenosine-rich series of bases prior to the IGF-IT polypeptide-specifying sequences. This construction is then inserted into an Xbal / Sall treated pBR-derived plasmid (pINT-γ-TXb4) at a manufactured Xbal site following the trp promoter/regulator sequence.

The resulting vector, designated pADP1, is employed as an expression vector in an E.coli host to generate a polypeptide product, IGF-IT7, including a "pro" sequence of 8 amino acids as follows: $NH_2$-$Met^{-8}$-$Lys^{-7}$-$Lys^{-6}$-$Tyr^{-5}$-$Trp^{-4}$-$Ile^{-3}$-$Pro^{-2}$-$Met^{-1}$ [IGF-IT]-COOH.

Plasmid pADP1 was digested with EcoRI and SalI to excise a fragment containing the trp promoter/regulator followed by the sequence coding for IGF-IT7. This fragment was then cloned into EcoRI / XhoI -treated pCFM414, the temperature-sensitive copy number plasmid described in Example 4. The XhoI cohesive terminus of the plasmid is ligated with the SalI cohesive terminus of the gene fragment, thereby eliminating both restriction sites in the resulting plasmid vector, pADP215.

The following example relates to microbial expression of desired polypeptides of the invention.

## EXAMPLE 7

### A. Expression of IGF-I

Plasmid pT5-4-IGF-I (Example 4) was transformed into E.coli strain JM103, and grown in M9 media, with 0.4% glycerol, 500 $\mu$g/ml ampicillin at a spectrophotometric absorbance reading of 0.2 at 600 nm wavelength ($A_{600}$ = 0.2). Isopropylthiogalactoside (IPTG) was added to 1 m M to induce the production of the IGF-I polypeptide. The culture was harvested at $A_{600}$ = 1.3, and cells were sonicated and centrifuged. The resulting pellet was then solubilized in 6.0 M guanidine hydrochloric acid / 0.1 M dithiothreitol (HCl/DTT) to denature the protein. This solution was then passed over a Sephadex G25 column in 0.01% HCl to remove the guanidine and DTT and renatured with 5 m M oxidized DTT. A radioimmunoassay of the type available from Nichols Institute Diagnostics was employed to quantify the expression of the IGF-I polypeptide. Radioimmunoassay (RIA) results indicate a value of 2 micrograms per liter of culture at a concentration corresponding to a spectrophotometric reading of 1 at 600 nm wavelength (2 $\mu$g/ODI) in whole cells and less than 0.18 $\mu$g/ODI in renatured cell pellet material.

Plasmid pADP223 (Example 4) was transformed into E.coli strain JM103 and grown in L broth, with 500 $\mu$g/ml ampicillin at 28°C and shifted to 37°C at $A_{600}$ = 0.1. The culture was harvested at $A_{600}$ = 2.0. Cells were sonicated, centrifuged and the pellet solubilized in guanidine HCl/DTT. This solution was then passed over a Sephadex G25 column and renatured with oxidized DTT. RIA results indicate 5 $\mu$g/ODI of material in whole cells and less than 0.26 $\mu$g/ODI in renatured cell pellet material.

### B. Expression of 8-Galactosidase-IGF-I Fusion Peptide

Plasmid pADP201 (Example 6), was transformed into the E.coli bacterial strain JM103, and transformed host cells were grown in M9 media, with 0.4% glycerol and 25 $\mu$g/ml tetracycline. At $A_{600}$ = 0.2, IPTG was added to 1 m M to induce the production of $\beta$-galactosidase-IGF-IT. The culture was harvested at $A_{600}$ = 0.8. The cells were disrupted by sonication and centrifuged. All the $\beta$-galactosidase-IGF-IT was in the pellet. The cell pellet was suspended in guanidine HCl/DTT to denature the protein and treated with cyanogen bromide (CNBr) to cleave protein at methionines. After roto-evaporation to remove the CNBr, the protein was retreated with guanidine HCl/DTT, passed through a Sephadex desalting column to remove the guanidine and DTT, and then renatured with oxidized DTT. RIA results indicate a yield of 250 $\mu$g/ODI.

### C. Expression of IGF-IT7

Plasmid pADP1 (Example 5) was transformed into E.coli bacterial strain HB101 and cells were grown in minimal media containing 500 $\mu$g/ml ampicillin. Cells were induced for production of IGF-IT7 by addition of indolacrylic acid at $A_{600}$ = 0.5. Cells were harvested at $A_{600}$ = 1.0. Cells were thereafter disrupted by freezing and thawing in the presence of lysozyme. This was followed by treatment with deoxyribonuclease and centrifugation. RIA results indicate that the supernatant contained 1 $\mu$g/ODI IGF-IT7.

Plasmid pADP2I5 (Example 6) was transformed into E.coli bacterial strain JM103 and grown in L broth, with 500 $\mu$g/ml ampicillin at 28°C and shifted to 37°C at $A_{600}$ = 0.1. The culture was harvested at $A_{600}$ =

2.0. Cells were sonicated, centrifuged, and the pellet solubilized in guanidine HCl/DTT. This material was then passed over a Sephadex G25 column, and renatured with oxidized DTT. RIA results indicate 20 μg/ODl of IGF-IT7 in whole cells and 250 μg/ODl of IGF-IT7 in the renatured cell pellet material.

Data on the expression systems is shown in Table IV below.

TABLE IV

| Expression System | ug/OD Liter | | |
|---|---|---|---|
| | Whole Cells | Supernatant | Pellet |
| pADP201 | 0.1 | -- | 250 |
| pADP1 | -- | 1 | -- |
| pADP215 | 20 | -- | 250 |
| pT5-4-IGF-I | 2 | -- | <0.18 |
| pADP223 | 5 | -- | <0.26 |

EXAMPLE 8

Biological Activity of IGF-IT7 and IGF-IT

To assess the possible influence of the eight amino acid leader on activity, lyophilized, HPLC-purified IGF-IT7 (100 μg) produced as described above was dissolved in 0.5 ml 70% formic acid containing 14 mg cyanogen bromide. After 64 hours at 23°C, the reaction mixture was passed through a 10 ml Sephadex G25 column which had been equilibrated with 1 $\underline{M}$ HCl. After the eluate was purified by HPLC, the protein was observed to have the same electrophoretic mobility as natural human plasma-derived IGF-I on PAGE, and was compared with IGF-IT7 and human plasma-derived IGF-I prepared according to Van Wyk, et al., PNAS (USA), 81 : 740-742 (1984) in the following assays:

A. A radioimmunoassay was performed by the non-equilibrium procedure of Copeland, et al., J.Clin. Endocrin.Metab., 50 : 690-697 (1980). The radioimmunoassay revealed that IGF-IT7 is 50% as potent as human-derived IGF-I; and minus the leader, as described above, the IGF-IT is 80% as potent as human-derived IGF-I.

B. A radioreceptor assay using human placental cell membranes was performed according to D'Ercole, et al., pages 190-201 in Growth Hormones and Related Peptides (Pecile, et al., eds.), Excerpta Medica, Amsterdam (1976). IGF-IT7 was observed to be 40% as potent as human-derived IGF-I and the IGF-IT was 80% as potent as the human standard.

C. In a cell growth assay to measure mitogenic activity according to Stiles, et al., J.Cell.Physiol., 99 : 395-406 (1979), both IGF-IT7 and IGF-IT stimulate further growth of BALB/C 3T3 cells. In nutritionally deprived medium after amino acid repletion, IGF-IT stimulates growth to the same extent as did the human IGF-I.

The foregoing illustrative examples are principally directed to construction of manufactured structural genes for IGF-I, IGF-II and analog polypeptides which are suited for use in direct expression and fusion product expression in E.coli host cells. Manufactured genes could readily be constructed in a manner especially well suited for direct expression in yeast cells through use of yeast preference codons. Further, the procedures of co-owned, co-pending U.S. Patent Application Serial No. 487,753, filed April 22, 1983, by Bitter and entitled "Secretion of Exogenous Polypeptides from Yeast" could be employed to secure yeast expression coupled with secretion of polypeptide products into the growth medium. In such a case it would be unnecessary to provide a methionine-specifying, translation initiating ATG codon 5' to the IGF-specifying codons.

Products of the present invention and/or antibodies thereto may be suitably "tagged", for example radiolabelled (e.g., with I[125]) conjugated with enzymes or fluorescently labelled, to provide reagent materials useful in assays and/or diagnostic test kits, for the qualitative and/or quantitative determination of the presence of such products and/or said antibodies in fluid samples. Such antibodies may be obtained from the inoculation of one or more animal species (e.g., mice, rabbit, goat, human, etc.) or from monoclonal antibody sources. Any of such reagent materials may be used alone or in combination with a suitable substrate, e.g., coated on a glass or plastic particle or bead.

Numerous modifications and variations in the practice of the invention are expected to occur to those skilled in the art upon consideration of the foregoing illustrative examples. Consequently, the invention should be considered as limited only to the extent reflected by the appended claims.

The features disclosed in the foregoging description, in the following claims and/or in the accompanying drawings may, both separately and in any combination thereof, be material for realising the invention in diverse forms thereof.

Plasmid pCFM414 was deposited under number A.T.C.C. 40074 with the American Type Culture Collection on 21 July 1983.

## Claims

1. A manufactured gene capable of directing the synthesis in bacteria of an insulin-like growth factor II polypeptide.

2. A manufactured gene according to claim 1 wherein the base sequence includes one or more codons, selected from among alternative codons specifying the same amino acid, on the basis of preferential expression characteristics of the codon in bacteria.

3. A manufactured gene according to claim 1 wherein the base sequence includes one or more codons, selected from among alternative codons specifying the same amino acid, on the basis of preferential expression characteristics of the codons in E.coli .

4. A manufactured gene specifying insulin-like growth factor I wherein the base sequence comprises the following: ·

```
5'-GGT CCG GAA ACA CTG TGC GGC GCT GAA CTG GTT GAC GCT
3'-CCA GGC CTT TGT GAC ACG CCG CGA CTT GAC CAA CTG CGA
CTG CAG TTT GTA TGC GGC GAC CGT GGT TTC TAT TTT AAC AAG
GAC GTC AAA CAT ACG CCG CTG GCA CCA AAG ATA AAA TTG TTC
CCG ACT GGC TAC GGT TCC TCT TCC CGC CGT GCT CCA CAG ACT
GGC TGA CCG ATG CCA AGG AGA AGG GCG GCA CGA GGT GTC TGA
GGT ATC GTT GAT GAA TGC TGT TTT CGT TCT TGC GAT CTG CGC
CCA TAG CAA CTA CTT ACG ACA AAA GCA AGA ACG CTA GAC GCG
CGT CTG GAA ATG TAT TGT GCT CCT CTG AAA CCC GCA AAG TCC
GCA GAC CTT TAC ATA ACA CGA GGA GAC TTT GGG CGT TTC AGG
GCT TAG TAG-3'
CGA ATC ATC-5'.
```

5. A manufactured gene specifying insulin-like growth factor II wherein the base sequence comprises the following:

```
5'-GCA TAT AGA CCA TCT GAA ACA CTG TGC GGC GGT GAA CTG
3'-CGT ATA TCT GGT AGA CTT TGT GAC ACG CCG CCA CTT GAC
GTT GAC ACT CTG CAG TTT GTA TGC GGC GAC CGT GGT TTC TAT
CAA CTG TGA GAC GTC AAA CAT ACG CCG CTG GCA CCA AAG ATA
TTT AGC CGT CCG GCT TCT CCC GTA TCC CGT CGC TCT CGT GGT
AAA TCG GCA GGC CGA AGA GCG CAT AGG GCA GCG AGA GCA CCA
ATC GTT GAG GAA TGC TGT TTT CGT TCT TGC GAT CTG GCT CTG
TAG CAA CTC CTT ACG ACA AAA GCA AGA ACG CTA GAC CGA GAC
CTG GAA ACC TAT TGT GCT ACT CCC GCA AAG TCC GAA TAG TAG-3'
GAC CTT TGG ATA ACA CGA TGA GGG CGT TTC AGG CTT ATC ATC-5'
```

6. A manufactured gene according to claim 1 wherein base codons specifying insulin-like growth factor include initial and/or terminal codons respectively specifying in substitution for or in addition to a codon for [Met$^{-3}$], additional initial and/or terminal amino acids in the polypeptide synthesized.

7. A manufactured gene according to claim 6 wherein said initial codons specifying additional initial amino acids include a codon specifying an initial methionine residue.

8. A manufactured gene according to claim 6 wherein said initial codons specifying additional initial amino acids are adinine-rich initial codons.

14

9. A manufactured gene according to claim 8 wherein said adenine-rich initial codons comprise the following sequence of bases:

5′ ATG AAG AAA TAT TGG ATC CCA-3′.

10. A manufactured gene according to claim 1 wherein the base codons specifying insulin-like growth factor are preceded and/or followed by and/or include a sequence of bases comprising a portion of a base sequence which provides a recognition site for restriction endonuclease enzyme cleavage.

11. A manufactured gene capable of directing the synthesis in a selected host microorganism of an insulin-like growth factor polypeptide analog which differs therefrom and from a [Met$^{-1}$] analog thereof in terms of the identity and/or location of one or more amino acids.

12. A manufactured gene according to claim 11 wherein the base sequence includes one or more codons, selected from among alternative codons specifying the same amino acid, on the basis of preferential expression characteristics of the codon in a projected host microorganism.

13. A manufactured gene according to claim 12 wherein the base sequence includes one or more codons, selected from among alternative codons specifying the same amino acid, on the basis of preferential expression characteristics of the codon in E.coli .

14. A manufactured gene according to claim 11 wherein base codons specifying insulin-like growth factor include initial and/or terminal codons respectively specifying additional initial and/or terminal amino acids in the polypeptide synthesized.

15. A manufactured gene according to claim 14 wherein said initial codons specifying additional initial amino acids are adenine-rich initial codons.

16. A manufactured gene according to claim 15 wherein said adenine-rich initial codons comprise the following sequence of bases:

5′ ATG AAG AAA TAT TGG ATC CCA-3′.

17. A manufactured gene according to claim 11 wherein the base codons specifying insulin-like growth factor are preceded and/or followed by and/or include a sequence of bases comprising a portion of a base sequence which provides a recognition site for restriction endonuclease cleavage of a DNA sequence.

18. A fusion gene comprising a manufactured gene according to claim 1 or 11 fused to a second gene capable of directing synthesis of a second polypeptide in a manner permitting the synthesis of a fused polypeptide including the polypeptide products coded for by the manufactured genes of claim 1 or 8.

19. A fusion gene according to claim 18 wherein said second gene is a gene directing synthesis of β-galactosidase enzyme.

20. A biologically functional DNA microorganism transformation vector including a manufactured gene according to claim 1 or 11.

21. A biologically functional DNA microorganism transformation vector including a fusion gene according to claim 18.

22. A vector according to either of claims 20 or 21 which is a circular DNA plasmid.

23. A microorganism transformed with a vector according to either of claims 20 or 21.

24. A process for the production of insulin-like growth factor polypeptide comprising:
growing, under appropriate nutrient conditions, microorganisms transformed with a biologically functional DNA including a manufactured gene according to claim 1, whereby said microorganisms express said gene and produce insulin-like growth factor polypeptide.

25. A process for the production of insulin-like growth factor polypeptide comprising:
growing, under appropriate nutrient conditions, microorganisms transformed with a biologically functional DNA including a manufactured gene according to claim 1, whereby said microorganisms express said gene and produce insulin-like growth factor polypeptide.

26. A process for the production of insulin-like growth factor polypeptide comprising:
growing, under appropriate nutrient conditions, microorganisms transformed with a biologically functional DNA including a manufactured gene according to claim 1, whereby said microorganisms express said gene and produce insulin-like growth factor polypeptide.

27. A process according to claim 24 or 25 wherein the microorganisms grown are E.coli microorganisms.

28. A process for the production of insulin-like growth factor polypeptide analog comprising:
growing, under appropriate nutrient conditions, microorganisms transformed with a biologically functional DNA including a manufactured gene according to claim 11, whereby said microorganisms express said gene and produce insulin-like growth factor polypeptide.

29. A process according to claim 28 wherein the microorganisms grown are E.coli microorganisms.

30. A polypeptide product of the expression in a microorganism of a manufactured gene according to claim 1.

31. A polypeptide product of the expression in a microorganism of a manufactured gene accordig to claim 11.

32. A reagent material comprising a radio-labelled manufactured gene according to claim 1 or 11.

33. A reagent material comprising a radio-labelled polypeptide product of claim 30 or 31.

34. A reagent material according to claim 32 or 33 wherein said radiolabel is $I^{125}$.

35. A reagent material comprising a tagged antibody to a polypeptide according to claim 30 or 31 coated on the surface of a plastic bead.

36. A manufactured gene, wherein base codons specifying insulin-like growth factor I include initial and/or terminal codons respectively specifying, in substitution for or in addition on to a codon for [Met$^{-1}$] terminal amino acids in the polypeptide synthesized.

37. A manufactured gene according to Claim 36, wherein said initial codons specifying additional initial amino acids include a codon specifying an initial methionine residue.

38. A manufactured gene according to Claim 36, wherein said initial codon specifying additional initial amino acids are adenine-rich initial codons.

39. A manufactured gene according to Claim 38, wherein said adenine-rich initial codons comprise the following sequence of bases:

5' ATG AAG AAA TAT TGG ATC CCA-3'

40. A fusion gene comprising a manufactured gene capable of directing the synthesis in a selected host microorganism of an insulin-like growth factor I polypeptide fused to a second gene capable of directing synthesis of a second polypeptide in a manner permitting the synthesis of a fused polypeptide including the polypeptide products coded for by the manufactured as genes of IGF-I manufactured gene such as the gene of Claim 8.

41. A fusion gene according to Claim 18 wherein said second gene is a gene directing synthesis of b-galactosidase enzyme.

European Patent Office

# EUROPEAN SEARCH REPORT

Application Number

EP 90 11 5259

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | BIOLOGICAL ABSTRACTS, RRM no. 26023523, Bioscience Information Services, Philadelphia, PA, US; G.T. MULLENBACH et al.: "The chemical synthesis molecular cloning and expression in yeast of genes coding for human insulin-like growth factors I and II", & FEDERATION PROCEEDINGS (USA), 1983, vol. 42, no. 7, abstract 434 | 4,5,20 | C 12 N    15/00<br>C 07 H    21/04<br>C 12 P    21/02<br>C 12 N    1/20  //<br>(C 12 N    1/20<br>C 12 R    1:19 ) |
| Y | IDEM | 1 | |
| E | NL-A-8 302 324  (RIJKSUNIVERSITEIT UTRECHT)<br>* Claims; fig. * | 4,20 | |
| E | EP-A-0 123 228  (CHIRON)<br>* Claims * | 4,5,20 | |
| Y | | 1 | |
| E | EP-A-0 128 733  (GENENTECH)<br>* Claims; fig. * | 4,20 | TECHNICAL FIELDS SEARCHED (Int. Cl.5) |
| E | EP-A-0 130 166  (KALIGEN AB)<br>* Claims * | 4,20 | C 12 N |
| E | WO-A-8 403 103  (PHARMACIA)<br>* Claims; page 28 * | 4,18,20 ,21 | |
| E | WO-A-8 500 829  (AMGEN)<br>* Claims; example 4 * | 4,20 | |

The present search report has been drawn up for all claims

| Place of search | Date of completion of the search | Examiner |
|---|---|---|
| THE HAGUE | 05-09-1990 | DELANGHE L. L. M. |